# EUROPEAN PATENT APPLICATION

(11) **EP 1 747 780 A1**
(43) Date of publication of application: **31.01.2007**
(21) Application number: 05741451.8
(22) Date of filing: 17.05.2005
(51) Int. Cl.: A61K 31/64, A61K 31/20, A61P 3/10, A61P 9/10, A61P 13/12

(54) **DRUG FOR PREVENTION OR TREATMENT OF DIABETES**

(30) Priority: 21.05.2004 US 573571 P
(71) Applicant: Kowa Company, Ltd., Nagoya-shi, Aichi 460-0003 (JP)
(72) Inventor: INOUE, Keisuke, 1970013 (JP); TAMAKI, Tarou, 1580094 (JP)
(74) Representative: Bannerman, David Gardner
(86) International application number: PCT/JP2005/008931
(87) International publication number: WO 2005/112944

(57) **Abstract**

The present invention provides a medicine for preventing or treating a diabetes, which includes 2,2-dichloro-12-(4-chlorophenyl)-dodecanoic acid or a pharmacologically acceptable salt thereof, and glibenclamide as active ingredients. The present invention provides a method for preventing or treating a diabetes, which includes administering 2,2-dichloro-12-(4-chlorophenyl)-dodecanoic acid or a pharmacologically acceptable salt thereof, and glibenclamide to a patient suffering from or having a possibility of suffering from diabetes.

## Description

### Technical Field

The present invention relates to a medicine for preventing or treating a diabetes, and more particularly to a medicine for preventing or treating a diabetes, which exhibits an excellent hypoglycemic effect.

### Background Art

Diabetes is a metabolic disorder caused by plural factors and is classified broadly into two types: type 1 diabetes caused by insulin hyposecretion; and type 2 diabetes caused by the decrease of insulin sensitivity in the peripheral tissue. Recently, type 2 diabetes is increasing rapidly due to environmental factors such as obesity and overeating. There are 7.4 million patients in Japan and 150 million patients in the world, and it is estimated that the patients will increase to 300 million by 2025. The diabetes patients have slight subjective symptoms in an early stage of diabetes. However, diabetes is an important risk factor of disorders relating to arteriosclerosis and is a cause of a diabetes complication such as diabetic nephropathy (suffered by 40% of dialysis patients) or diabetic retinopathy. Therefore, appropriate treatment and management are required. Type 2 diabetes is developed due to an insufficient insulin supply without being able to meet increase in insulin demand caused by failure in the function of insulin (insulin resistance). In order to treat type 2 diabetes, exercise therapy, dietary therapy, or medicinal therapy has been performed.

In medicinal therapy, a sulfonylurea agent, a biguanide agent, a glitazone drug, or the like is used in the clinical field (Non-Patent Document 1, Non-Patent Document 2). However, such medicines are disadvantageous in that they have insufficient hypoglycemic effect or in that the blood-sugar level decreases insufficiently due to a low-dose use for preventing the development of side effects.

Recently, a novel type of diabetes therapeutic agent such as a 2,2-dichloroalkane carboxylate compound has been developed (Non-Patent Document 3).
[Non-Patent Document 1] Silvio E. Inzucchi, JAMA 287, pp 360-372, 2002.
[Non-Patent Document 2] Eric S. Holmboe, JAMA 287, pp373-376, 2002.
[Non-Patent Document 3] Kirstin Meyer et al., European Journal of Medicinal Chemistry, 33, pp775-787, 1998.

### Disclosure of the Invention

### [Problems to be solved by the Invention]

It is an object of the present invention to provide a medicine for preventing or treating a diabetes, which has no side effect or the like and exhibits an excellent hypoglycemic effect.

### [Means for solving the Problems]

In view of such circumstances, the inventors of the present invention have made extensive studies. As a result, they have found out that an excellent hypoglycemic effect is exhibited when using 2,2-dichloro-12-(4-chlorophenyl)-dodecanoic acid or a pharmacologically acceptable salt thereof in combination with glibenclamide which is one of sulfonylurea agents, thereby achieving the present invention.

That is, the present invention provides a medicine for preventing or treating a diabetes, which includes 2,2-dichloro-12-(4-chlorophenyl)-dodecanoic acid or a pharmacologically acceptable salt thereof, and glibenclamide as active ingredients.

The medicine of the present invention for preventing or treating a diabetes may preferably be used for a prevention or a treatment of particularly type 2 diabetes.

Furthermore the medicine of the present invention for preventing or treating a diabetes is characterized in that the 2,2-dichloro-12-(4-chlorophenyl)-dodecanoic acid or a pharmacologically acceptable salt thereof, and the glibenclamide may be separately administered.

The present invention provides a medicine for preventing or treating a diabetes complication, which includes 2,2-dichloro-12-(4-chlorophenyl)-dodecanoic acid or a pharmacologically acceptable salt thereof, and glibenclamide as active ingredients.

Moreover, the medicine of the present invention for preventing or treating a diabetes complication may preferably be used for a prevention or a treatment of particularly diabetic nephropathy, diabetic retinopathy, diabetic neuropathy, arteriosclerosis, or the like.

Furthermore, the medicine of the present invention for preventing or treating a diabetes complication is characterized in that the 2,2-dichloro-12-(4-chlorophenyl)-dodecanoic acid or a pharmacologically acceptable salt thereof, and the glibenclamide may be separately administered.

The present invention provides a method for preventing or treating a diabetes, which includes administering 2,2-dichloro-12-(4-chlorophenyl)-dodecanoic acid or a pharmacologically acceptable salt thereof, and glibenclamide to a patient suffering from or having a possibility of suffering from diabetes.

Furthermore, the method of the present invention for preventing or treating a diabetes may preferably be used for a prevention or a treatment of particularly type 2 diabetes.

The present invention provides a method for preventing or treating a diabetes complication, which includes administering 2,2-dichloro-12-(4-chlorophenyl)-dodecanoic acid or a pharmacologically acceptable salt thereof, and glibenclamide to a patient suffering from or having a possibility of suffering from a diabetes complication.

Moreover, the method of the present invention for preventing or treating a diabetes complication may preferably be used for a prevention or a treatment of particularly diabetic nephropathy, diabetic retinopathy, diabetic neuropathy, arteriosclerosis, or the like.

### Best Mode for carrying out the Invention

2,2-Dichloro-12-(4-chlorophenyl)-dodecanoic acid or a pharmacologically acceptable salt thereof to be used in the present invention may be produced by the method described in US 5, 968, 982 or JP 10-510515 A (WO 96/15784). Specifically, 1,10-dibromodecane is allowed to react with 4-chlorophenyl magnesium bromide to yield 1-bromo-10-(4-chlorophenyl)-decane. Subsequently, the resultant compound is allowed to react with dichloroacetic acid in the presence of lithium diisopropylamide (LDA), to thereby produce 2,2-dichloro-12-(4-chlorophenyl)-dodecanoic acid. Meanwhile, a pharmacologically acceptable salt thereof may be produced by a general method.

Examples of the salt include: alkaline metal salts such as sodium salts and potassium salts; alkaline earth metal salts such as calcium salts and magnesium salts; and organic base salts such as ammonium salts and trialkylamine salts. Of those, the sodium salts are particularly preferred.

Glibenclamide to be used in the present invention is easily available from SIGMA-ALDRICH Corporation (trade name: Glybenclamide or Glyburide).

The medicine of the present invention includes 2,2-dichloro-12-(4-chlorophenyl)-dodecanoic acid or a pharmacologically acceptable salt thereof, and glibenclamide at a mass ratio ranging preferably from 1:20 to 80:1, particularly preferably from 1:10 to 32:1.

The medicine of the present invention can be mixed with additives used generally for manufacture of a medicine, in addition to the active ingredients. Examples of the additives include an excipient, an extender, a disintegrator, a binding agent, a lubricant, a diluent, a buffer agent, an antiseptic agent, an emulsifying agent, and a stabilizing agent.

Examples of the excipient or the extender include starches, lactose, sucrose, mannitol, and silicic acid.

Examples of the disintegrator include agar, calcium carbonate, potato or tapioca starch, alginic acid, and specific complex silicate.

Examples of the binding agent include carboxymethylcellulose, alginate, gelatin, polyvinyl pyrrolidone, sucrose, and gum arabic.

Examples of the lubricant include talc, calcium stearate, magnesium stearate, solid polyethylene glycols, sodium lauryl sulfate, and mixtures thereof.

Examples of the diluent include lactose and corn starch.

Examples of the buffer agent include: organic acids such as citric acid, phosphoric acid, tartaric acid, and lactic acid; inorganic acids such as hydrochloric acid; alkali hydroxides such as sodium hydroxide and potassium hydroxide; and amines such as triethanolamine, diethanolamine, and diisopropanolamine.

Examples of the antiseptic agent include paraoxybenzoates and benzalkonium chloride.

Examples of the emulsifying agent include: anionic surfactants such as calcium stearate, magnesium stearate, and sodium lauryl sulfate; cationic surfactants such as benzalkonium chloride, benzethonium chloride, and cetyl pyridinium chloride; and nonionic surfactants such as glyceryl monostearate, sucrose fatty acid ester, polyoxyethylene hydrogenated castor oil, polyoxyethylene sorbitan fatty acid ester, polyoxyethylene fatty acid ester, and polyoxyethylene alkyl ether.

Examples of the stabilizing agent include sodium sulfite, sodium bisulfite, dibutylhydroxytoluene, butylhydroxyanisole, and edetic acid.

The medicine of the present invention can be supplied in various dosage forms such as a tablet, a capsule, a granule, and a film-coating agent according to its usage.

As the medicine of the present invention, the two active ingredients may be orally administered at the same time as one preparation or as separate preparations. In addition,the two active ingredients may be orally administered separately at intervals.

Therefore, the medicine of the present invention may be a combination drug which is obtained by combining 2,2-dichloro-12-(4-chlorophenyl)-dodecanoic acid or a pharmacologically acceptable salt thereof, and glibenclamide. Additionally, the medicine of the present invention may be pharmaceutical packs or kits comprising a medicine containing 2,2-dichloro-12-(4-chlorophenyl)-dodecanoic acid or a pharmacologically acceptable salt thereof, and a medicine containing glibenclamide.

The dose of the medicine of the present invention is arbitrarily selected depending on the weight, age, sex, symptom, or the like of the patient. In the case of an adult, it is suitable that 2,2-dichloro-12-(4-chlorophenyl)-dodecanoic acid or a pharmacologically acceptable salt thereof be administered in an amount of generally 1 to 80 mg, preferably 1 to 40 mg per day. Meanwhile, it is suitable that glibenclamide be administered in an amount of 1 to 20 mg, preferably 1.25 to 10 mg per day. Moreover, the administration may be performed once a day or may be performed twice or more per day.

Next, the present invention will be described in more detail by way of examples, but the present invention is not limited to the examples.

### Examples

The hypoglycemic effect of single administration or combined administration of sodium 2,2-dichloro-12-(4-chlorophenyl)-dodecanoate (synthesized by the aforementioned method) and glibenclamide was determined by the following method (Metabolism, 48, pp34-40, 1999, Journal of Medicinal Chemistry, 44, pp2601-2611, 2001). As test animals, C57BL/KsJ db/db mice were used, which were created in Jackson Laboratory (USA) and known as an obesity, hyperlipemia, hyperinsulinemia, and insulin-resistant model (Journal of Clinical Investigation, 85, pp962-967, 1990).

Blood was collected from the orbital venous plexus of each 7-week-old db/db mouse using a heparin-treated capillary tube, and centrifugation was performed to collect plasma. Thereafter, the plasma glucose concentration (Glucose CII-Test Wako (Wako Pure Chemical Industries, Ltd.)), the insulin concentration (Lebis Insulin Kit: for mouse-T (SHIBAYAGI)), and the triglyceride concentration (Triglyceride E-Test Wako (Wako Pure Chemical Industries, Ltd.)) were measured for classification. The classification was performed so that distribution of each measurement item is uniform for each group by block allocation based on many variables in which the plasma glucose concentration is most emphasized using the measured values of the plasma glucose concentration, the body weight, the insulin concentration, and the triglyceride concentration.

The medicines were administered as follows: to the sodium 2,2-dichloro-12-(4-chlorophenyl)-dodecanoate single administration group, sodium 2,2-dichloro-12-(4-chlorophenyl)-dodecanoate (3 mg/kg: 0.09 to 0.12 mg/body (individual)) was orally administered singly once a day from the next day of the blood collection to the 14th day; and to the glibenclamide single administration group, glibenclamide (30 mg/kg: 1.00 to 1.18 mg/body (individual)) was orally administered singly once only on the 14th day from the next day of the blood collection. Meanwhile, to the sodium 2,2-dichloro-12-(4-chlorophenyl)-dodecanoate/glibenclamide combined administration group, sodium 2,2-dichloro-12-(4-chlorophenyl)-dodecanoate (3 mg/kg: 0.09 to 0. 14 mg/body (individual)) was orally administered once a day until the 13th day, and both medicines (sodium 2,2-dichloro-12-(4-chlorophenyl)-dodecanoate (3 mg/kg: 0.09 to 0.14 mg/body (individual)) and glibenclamide (30 mg/kg: 1.07 to 1.40 mg/body (individual))) were orally administered once only on the 14th day.

Two hours after the administration, blood was collected from the orbital venous plexus, and the plasma thereof was collected to measure the plasma glucose concentration.

Table 1 shows plasma glucose concentrations on the 14th day after administration for the sodium 2,2-dichloro-12-(4-chlorophenyl)-dodecanoate single administration group, the glibenclamide single administration group, and the both medicines combined administration group. Each plasma glucose concentration is expressed as mean ± standard deviation for 6 mice of each group. Each decreasing rate is calculated from ((mean of plasma glucose concentration of control group) - (mean of plasma glucose concentration of each group)) / (mean of plasma glucose concentration of control group) × 100, while each relative index is calculated from (mean of plasma glucose concentration of each group) / (mean of plasma glucose concentration of control group).

As a result, in the case of single administration of sodium 2,2-dichloro-12-(4-chlorophenyl)-dodecanoate, the plasma glucose concentration decreased by 51%, but the level was still higher than that of a db/+m mouse (187 ± 16), which is considered as a normal mouse in contrast with the model mouse. Meanwhile, in the case of single administration of glibenclamide, the plasma glucose concentration decreased only by 21% . On the other hand, in the case of combined administration of the both medicines, the plasma glucose concentration decreased to a normal plasma glucose concentration, and the relative index (0.32) was smaller than the product (0.39) of the relative indices of the respective single administration groups, so that the synergistic effect due to combination was confirmed.

**Table 1**

| Test medicine | Plasma glucose concentration (mg/dl) | Decreasing rate | Relative index |
|---|---|---|---|
| Control group | 519±69 | | |
| Sodium 2,2-dichloro-12-(4-chlorophenyl)-dodecanoate single administration group (3 mg/kg) | 253±75 | 51% | 0.49 |
| Glibenclamide single administration group (30 mg/kg) | 412±153 | 21% | 0.79 |
| Both medicines combined administration group | 166±42 | 68% | 0.32 |

Each plasma glucose concentration is expressed as mean ± standard deviation for 6 mice of each group.

### Industrial Applicability

A medicine of the present invention for preventing or treating a diabetes has no side effect or the like and exhibits an excellent hypoglycemic effect, so that it is useful in preventing or treating a diabetes and a diabetes complication.

## Claims

1. A medicine for preventing or treating a diabetes, which comprises 2,2-dichloro-12-(4-chlorophenyl)-dodecanoic acid or a pharmacologically acceptable salt thereof, and glibenclamide as active ingredients.

2. A medicine for preventing or treating a diabetes according to claim 1, wherein the diabetes is type 2 diabetes.

3. A medicine for preventing or treating a diabetes according to claim 1, wherein the 2,2-dichloro-12-(4-chlorophenyl)-dodecanoic acid or a pharmacologically acceptable salt thereof, and the glibenclamide are separately administered.

4. A medicine for preventing or treating a diabetes complication, which comprises 2,2-dichloro-12-(4-chlorophenyl)-dodecanoic acid or a pharmacologically acceptable salt thereof, and glibenclamide as active ingredients.

5. A medicine for preventing or treating a diabetes complication according to claim 4, wherein the diabetes complication is selected from diabetic nephropathy, diabetic retinopathy, diabetic neuropathy, and arteriosclerosis.

6. A medicine for preventing or treating a diabetes complication according to claim 4, wherein the 2,2-dichloro-12-(4-chlorophenyl)-dodecanoic acid or a pharmacologically acceptable salt thereof, and the glibenclamide are separately administered.

7. A method for preventing or treating a diabetes, which comprises administering 2,2-dichloro-12-(4-chlorophenyl)-dodecanoic acid or a pharmacologically acceptable salt thereof, and glibenclamide to a patient suffering from or having a possibility of suffering from diabetes.

8. A method for preventing or treating a diabetes according to claim 7, wherein the diabetes is type 2 diabetes.

9. A method for preventing or treating a diabetes complication, which comprises administering 2,2-dichloro-12-(4-chlorophenyl)-dodecanoic acid or a pharmacologically acceptable salt thereof, and glibenclamide to a patient suffering from or having a possibility of suffering from a diabetes complication.

10. A method for preventing or treating a diabetes complication according to claim 9, wherein the diabetes complication is selected from diabetic nephropathy, diabetic retinopathy, diabetic neuropathy, and arteriosclerosis.
